# EUROPEAN PATENT APPLICATION

(11) **EP 0 717 995 A2**
(43) Date of publication of application: **26.06.1996**
(21) Application number: 95115941.7
(22) Date of filing: 10.10.1995
(51) Int. Cl.: A61K 31/725

(54) **Cell degeneration suppressing and organ toxicity reducing agent**

(30) Priority: 11.10.1994 JP 245233/94
(71) Applicant: SANWA KAGAKU KENKYUSHO CO., LTD., Higashi-ku Nagoya-shi Aichi-ken 461 (JP)
(72) Inventor: Sawai, Kiichi, c/o Sanwa Kagaku Kenkyusho Co., Nagoya-shi, Aichi (JP); Mitani, Takahiko, c/o Sanwa Kagaku Kenkyusho Co., Nagoya-shi, Aichi (JP); Ishiwata, Yoshiro, c/o Sanwa Kagaku Kenkyusho Co., Nagoya-shi, Aichi (JP); Tomiya, Noboru, c/o Sanwa Kagaku Kenkyusho Co., Nagoya-shi, Aichi (JP); Usui, Toshinao, c/o Sanwa Kagaku Kenkyusho Co., Nagoya-shi, Aichi (JP)
(74) Representative: Weisert, Annekäte, Dipl.-Ing. Dr.-Ing.

(57) **Abstract**

This invention provides an agent for use in the suppression of cell degeneration and reduction of organ toxicity, which comprises a sulfated glycolipid or fucosylated glycolipid of biological origin, a synthetic sulfated saccharide, or a mixture composition thereof as an inhibitor for a neutrophil surface-adhering molecule selectin. The agent of the present invention is useful as a preventive and therapeutic agent of cell degeneration and organ disorders caused by poisoning accidents and viral infection and can reduce side effects of drugs for use in the treatment of causative diseases, so that such drugs can be administered safely to patients who are apt to receive side effects due to reduced functions in the liver, kidney and the like organs, cerebral tissues and cells or who have a narrow range of the application dose of medicines like the case of pregnant women, babies, the aged and the like.

## Description

### FIELD OF THE INVENTION

This invention relates to an agent for use in the suppression of cell degeneration and reduction of organ toxicity, which contains as the main ingredient an inhibitor for a neutrophil surface-adhering molecule selectin.

### BACKGROUND OF THE INVENTION

When abnormality occurs in tissues of the living body, intravascular leukocytes rapidly approach to the abnormal tissue by starting adhesion to endothelial cells and migrating through the cells into the extravascular area. It is known that selectin (types E, P and L) is deeply concerned in this process as a leukocyte-adhering receptor and acts as a factor inducing vascular wall inflammatory disorders depending on the degree of adhesion to endothelial cells.

Though it may not be certain without further studies that inhibition of selectin family always produces good results in the living body, because not only selectin but also integrin family and immunoglobulin family are related to the leukocyte receptor-adhering factor, various biologically-related substances have been isolated and purified in recent years as inhibitors for the neutrophil surface-adhering molecule (i.e. selectin) and their anti-inflammatory actions have been reported.

In addition, attention has also been focused in recent years on sulfated polysaccharides as compounds having selectin inhibition activity (WO-9414849, WO-9414851).

As inhibitors of the neutrophil surface-adhering molecule, namely selectin, biologically-related substances have been screened by the inventors of the present invention. Though studies on these substances revealed a possibility of using them as preventive or therapeutic agents of vascular wall inflammatory disorders, as well as their use in the treatment of ARDS, sepsis, shock, rheumatism, nephritis and the like, most of them did not satisfy desired conditions including high safety, large scale productivity and high preservation stability for the practical medicine.

Among sulfated glycolipids, the present inventors focused on sulfatides, sialyl Lewis compounds and commercially available sulfated polysaccharides, as substances which have high selectin (types E, P and L) inhibiting activity and which can be obtained in relatively large quantity in the living body. The inventors of the present invention have further conducted studies on the method for combined administration of these substances and their application to pharmaceutical preparations and carried out screening of their drug effects. Also, studies on the large scale production of a limited number of high molecular substances which are present in the living body in trace amount are now in progress making use of genetic engineering techniques.

Results of the screening of drug effects revealed that selectin inhibitors are deeply concerned not only in tissue damage in the living body but also in biological abnormality caused by abnormality-inducing substances (poisons and medicines) and parasites, and the behavior of intravascular leukocytes to approach rapidly to abnormal tissues is also triggered, for example, at the time of gas poisoning and food poisoning, at the time of the administration of anticancer drugs and general medicines considered to be necessary for apothanasia and, depending on physical constitution, even by the partake of substances which can be regarded as food, thus indicating that the selectin family is deeply concerned in food allergy and diseases which are considered to be side effects of medicines (organ disorders, anaphylaxis and the like).

Since not only selectin but also integrin family and immunoglobulin family are related to the leukocyte receptor adhering factor as described in the foregoing, there may be a necessity to conduct studies from the viewpoint of their mutual relation. In any case, the inventors of the present invention have confirmed that certain selectin inhibitors have effects to prevent side effects of medicines, to suppress toxic substance-induced degeneration of normal cells and to reduce organ toxicity, thereby accomplishing the present invention. The present inventors have also found that the selectin family takes part even in a changing process to a morbid state of hepatitis B or C in which tissue cells are put under a chronic abnormal state to repeat damage and regeneration many times, and finally hepatic cirrhosis or liver cancer occur. Thus, the pharmaceutical preparation of the present invention also provides suppressing effect of such disorders.

### SUMMARY OF THE INVENTION

Thus, the present invention provides:
1. an agent for suppressing cell degeneration and reducing organ toxicity, which comprises as a main ingredient an inhibitor for a neutrophil surface-adhering molecule selectin;
2. an agent for suppressing cell degeneration and reducing organ toxicity, wherein the selectin inhibitor, which comprises a sulfated glycolipid or fucosylated glycolipid of biological origin, a synthetic sulfated saccharide, or a mixture composition thereof, in which
   the sulfated glycolipid of biological origin is preferably selected from (1) sulfatide, (2) seminolipid, (3) SM3 (II³ SO₃ Lac Cer), (4) SB2 (III³ II³ SO₃ Ge3Cer) and (5) SB1a (IV³ SO₃ II³ SO₃ Ge4Cer),
   the fucosylated glycolipid is selected from (1) sialyl Lewis X (SLe^{x}) and (2) sialyl Lewis A (SLe^{a}), and
   the synthetic sulfated saccharide is selected from (1) chondroitin sulfate, (2) heparitin sulfate, (3) keratan sulfate, (4) dextran sulfate and (5) galactose sulfate.
3. an agent for suppressing cell degeneration and reducing organ toxicity, wherein the agent is used for the following purposes a) to e)
   a) prolonged administration of medicines having high toxicity,
   b) suppression of renal disorders caused by substances having renal toxicity,
   c) suppression of hepatic disorders caused by substances having hepatic toxicity,
   d) suppression of nervous disorders caused by substances having cranial nerve system toxicity, and
   e) reduction of organ disorders induced by microorganisms as the causative agent,
   preferably useful in
   (1) suppressing renal disorders caused by administration of indomethacin-based analgesics or kanamycin-based antibiotics,
   (2) suppressing hepatic and renal disorders caused by administration of 5-fluorouracil or cisplatin-based anticancer drugs,
   (3) suppressing nervous disorders caused by mercury or other heavy metal substances,
   (4) suppressing hepatic disorders caused by carbon tetrachloride or chronic alcoholism,
   (5) reducing hepatic disorders caused by infection with A type, B type, C type or non-A non-B non-C type hepatitis viruse, and
   (6) suppressing immunodeficiencies and cranial nerve disorders caused by infection with various retroviruses.

### DETAILED DESCRIPTION OF THE INVENTION

According to the present invention, there is provided an agent for suppressing cell degeneration and reducing organ toxicity, which comprises a formulation of one or more of the following natural or synthetic compounds:
a sulfated glycolipid selected from (1) sulfatide of the following formula 1, (2) seminolipid of the following formula 2, (3) SM3 (II³ SO₃ Lac Cer) of the following formula 3, (4) SB2 (III³ II³ SO₃ Ge3Cer) of the following formula 4 and (5) SB1a (IV³ SO₃ II³ SO₃ Ge4Cer) of the following formula 5, 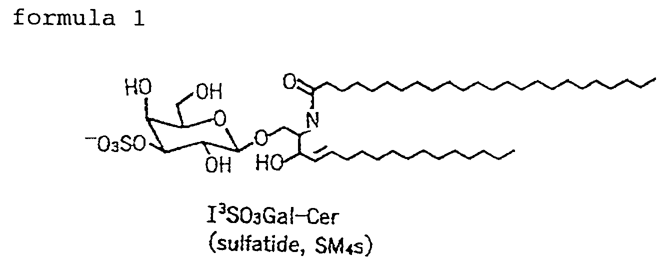 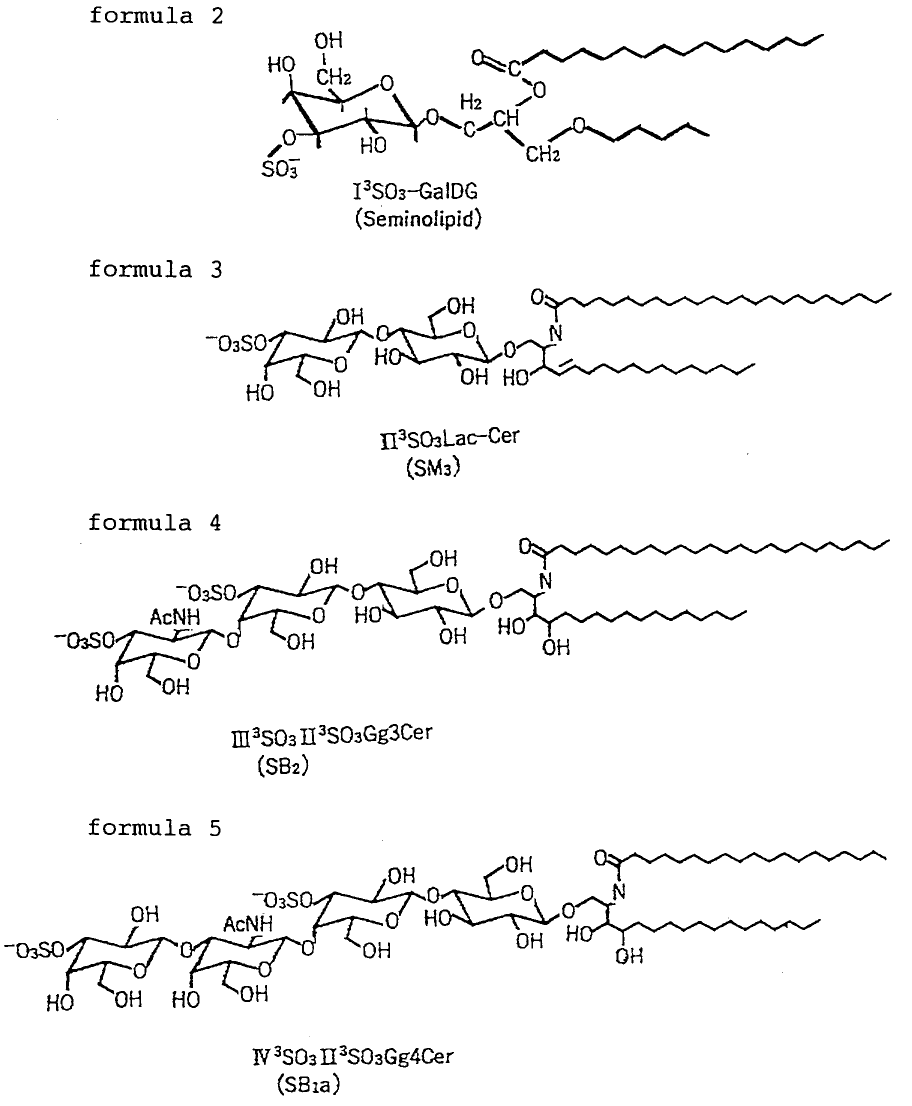
a fucosylated glycolipid of biological origin selected from (1) sialyl Lewis X (SLe^{x}) and (2) sialyl Lewis A (SLe^{a}), and a synthetic sulfated saccharide selected from (1) chondroitin sulfate, (2) heparitin sulfate, (3), keratan sulfate, (4) dextran sulfate and (5) galactose sulfate.

When the inhibitor according to the present invention for the neutrophil surface-adhering molecule selectin is practically administered to human, it is desirable to use the inhibitor as an action-activated composition in which an action activating and stabilizing carrier is contained in an amount of from 0.005 to 50% by weight based on 0.005 to 5% by weight of the compound of the present invention. Such a composition is effective in improving poisoning symptoms, preventing and treating microbial infection diseases such as hepatitis B, hepatitis C, wart, AIDS and the like, preventing side effects of medicines at the time of their long-term administration, particularly in preventing side effects of interferon and reducing its dose during interferon therapy, and preventing tissue damage and organ disorders at the time of the administration of anticancer drugs.

Examples of the action activating and stabilizing carrier include saccharides such as lactose, sucrose, dextrans and the like, cellulose-based polymer compounds such as hydroxypropylcellulose and the like and natural polymercompounds such as albumin and the like. In addition, such a composition may be made into mixture preparations by further adding generally used drugs having high direct therapeutic effects (for example, antiviral agents in the case of viral hepatitis, antiallergy agents in the case of allergic diseases and anticancer drugs in the case of cancers). In that case, significant therapeutic effects can be expected, because toxicity of the drugs having high direct therapeutic effects is reduced.

### (Dosage form and dose)

The inhibitor according to the present invention for the neutrophil surface-adhering molecule, selectin, can be used not only in usual dosage forms but also as enteric coated preparations in accordance with the characteristics of the drug to be blended. When the drug of the present invention is administered to human, its dose varies depending on the dosage form, age of each patient and the like, but may be within the range of from 1 to 1,500 mg. For example, it may be used in a dose of approximately from 1 to 5 mg/day per adult (50 kg in body weight) in the case of injection or of approximately 10 to 100 times larger than that in the case of oral administration.

The inhibitor of the neutrophil surface-adhering molecule selectin of the present invention can be used in the form of combination drugs together with medicines which show strong organ toxicity and tissue degeneration, such as antibiotics (penicillin-based, cephalosporin-based, kanamycin-based and streptomycin-based antibiotics), anticancer and antiviral drugs (mitomycin, 5-fluorouracil and cisplatin), narcotics, hypnotics, tranquilizers and the like. It is necessary to devise proper dosage form such as double layer tablets when the combination becomes an incompatibility drug. Even in the case of simple mixing, it is desirable to make a composition form in which the inhibitor of the neutrophil surface-adhering molecule, selectin, is adsorbed to a pharmaceutical carrier such as albumin or the like, in order to keep its activity and improve the absorption.

The following production examples, pharmacological drug effect test examples and drug preparation examples are provided to further illustrate the present invention.

### PRODUCTION EXAMPLE (SYNTHESIS)

### 3-O-(β-D-Galactopyranosyl 3-sulfate)-2-O-hexadecanoyl-1-O-hexadecyl-L-glycerol (seminolipid)

The title compound was synthesized in the following manner in accordance with the method of Roy Gigg (*J. Chem. Soc. Perkin I*, 1978, 712).

That is, a mixture of 93 g of 2-O-(buten-2-yl)-1-O-hexadecyl-L-glycerol, 250 g of tetra-O-acetylgalactopyranosyl bromide, 170 g of mercury(II) cyanide, 1.0 ℓ of benzene and 1.0 ℓ of nitromethane was stirred overnight at 40°C, diluted with 2.0 ℓ of benzene and washed with water and saturated sodium bicarbonate in that order, and the solvent was then evaporated under a reduced pressure. The thus obtained residue was added to a mixture of 200 g of sodium hydroxide, 2.5 ℓ of methanol and 100 ml of water, heated under reflux for 30 minutes and then neutralized with dry ice while stirring on an ice bath, followed by evaporation of the solvent under a reduced pressure. The resulting residue was mixed with ethyl acetoacetate and vigorously stirred, and then insoluble materials were removed by filtration and the solvent was evaporated under a reduced pressure.

After addition of a 25 g portion of the thus obtained residue to a mixture of 5.0 g of p-toluene sulfonate and 1.0 ℓ of acetone and subsequent 6 hours of stirring at room temperature, 10 ml of triethylamine and 5.0 g of sodium bicarbonate were added thereto and the solvent was evaporated under a reduced pressure. An ether extract from the resulting residue was purified by an alumina column chromatography to obtain 100 g of 2-O-(buten-2-yl)-1-O-hexadecyl-3-O-(3,4-O-isopropylidene-β-D-galactopyranosyl)-L-glycerol. A 6.0 g portion of the thus obtained compound was mixed with 6.0 g of 60% sodium hydride, 7.0 g of benzyl bromide and 100 ml of N,N-dimethylformamide, and the mixture was stirred overnight at room temperature, poured into ice water and then extracted with ether. The extract was dried over potassium carbonate and the solvent was evaporated under a reduced pressure.

The thus obtained residue was purified by silica gel column chromatography and then, together with 120 ml of 1 N hydrochloric acid, heated under reflux for 15 minutes. After cooling, sodium bicarbonate was added thereto, the solvent was evaporated under a reduced pressure, the resulting residue was extracted with ether and then the solvent was evaporated under a reduced pressure.

The thus obtained residue was mixed with 3.0 g of di-n-butyltin oxide and benzene, and the mixture was heated under reflux for 5 hours, simultaneously effecting fractional distillation of the generated water. Benzene was evaporated under a reduced pressure, and the thus obtained residue was mixed with 50 ml of N,N-dimethylformamide and 2.5 g of allyl bromide and stirred overnight at 100°C.

The reaction solution was poured into ice water, extracted with ether and then dried over potassium carbonate. The solvent was evaporated under a reduced pressure, and the residue was purified by an alumina column chromatography. A 10 g portion of the thus obtained compound was mixed with 5.0 g of 60% sodium hydride, 8.5 g of benzyl bromide and 100 ml of N,N-dimethylformamide, and the mixture was stirred overnight at room temperature, poured into ice water and then extracted with ether.

The extract was dried over potassium carbonate and the solvent was evaporated under a reduced pressure. A 5.4 g portion of the thus obtained residue was added to dimethyl sulfoxide solution containing 6.0 g t-butoxy potassium, and the mixture was subjected to 5 hours of reaction at 50°C, poured into ice water, extracted with ether and then dried over potassium carbonate. After evaporation of the solvent under a reduced pressure, the resulting residue was purified by alumina column chromatography, dissolved in 40 ml of pyridine, mixed with 1.9 g of hexadecanoyl chloride and then the mixture was stirred for 5 hours at room temperature.

The reaction solution was poured into ice water and extracted with ether and then the solvent was evaporated under a reduced pressure. The residue was added to a mixture solution of 150 ml of acetone, 4.5 g of mercuric chloride and 10 ml of water, the resulting mixture was stirred for 1 hour at room temperature and then the solvent was evaporated under a reduced pressure. The thus obtained residue was mixed with ether and washed with saturated potassium iodide aqueous solution and saturated potassium chloride aqueous solution.

After evaporation of the solvent under a reduced pressure, the resulting residue was purified by alumina column chromatography, and the purified product was dissolved in 50 ml of pyridine, mixed with 4.5 g of pyridine·sulfur trioxide, subjected to 5 hours of reaction at 50°C and then diluted with ether.

After washing with water, 2 N hydrochloric acid and saturated sodium carbonate in that order, the solvent was evaporated under a reduced pressure. The residue was mixed with 100 ml of acetic acid and a catalytically effective amount of 10% palladium carbon, and the mixture was stirred for 2 days at room temperature in an atmosphere of hydrogen gas. After removing insoluble materials by filtration, the solvent was evaporated under a reduced pressure to obtain the title compound.

### COMPOSITION PRODUCTION EXAMPLE

One part of albumin and 2 parts of the compound of the present invention (sulfatide, sialyl Lewis X or galactose sulfate) were kneaded using ethanol as the wetting agent and then dried under a reduced pressure to obtain a powder or granular composition.

### PREPARATION EXAMPLE 1

### Capsule preparation

A capsule preparation of the following formulation was prepared.

| | |
|---|---|
| Galactose Sulfate Obtained in Composition Production Example | 10.0 |
| Magnesium Stearate | 1.8 |
| Hydroxypropylcellulose | 2.7 |
| Lactose | proper amount |
| Total | 180.0 mg |

### PREPARATION EXAMPLE 2

### Injection preparation

An injection preparation of the following formulation was prepared.

| | |
|---|---|
| Selectin Inhibitor Obtained in Composition Production Example | 1.0 |
| Physiological Saline | proper amount |
| Total | 10.0 ml |

### PHARMACOLOGICAL DRUG EFFECT TEST EXAMPLE 1 (PREVENTIVE ACTION AGAINST NEPHRITIS)

Protective effect of the pharmaceutical preparation of the present invention against spontaneous nephritis in MRL/1 mice was examined (protective effect on functional reduction due to aging).

### I) Drugs used

### a) Sulfated glycolipid of biological origin

(1) sulfatide, (2) seminolipid, (3) SM3 (II³ SO₃ Lac Cer), (4) SB2 (III³ II³ SO₃ Ge3Cer), (5) SB1a (IV³ SO₃ II³ SO₃ Ge4Cer)

### b) Fucosylated glycolipid of biological origin

(1) sialyl Lewis X (SLe^{x}), (2) sialyl Lewis A (SLe^{a})

### c) Synthetic sulfated saccharide

(1) chondroitin sulfate, (2) heparitin sulfate, (3) keratan sulfate, (4) dextran sulfate, (5) galactose sulfate
Of these samples tested, commercially available compounds were purchased, and the others were prepared in accordance with the method reported by the present inventors in *BIOCHEM. BIOPHY. RES. COM*., 1993, No.2, 426.

### II) Procedure

Each test sample of 1 to 10 mg/kg mixed with feed was orally administered to male MRL/1 mice of 6 weeks of age (15 animals per 1 group) twice a week for 12 weeks. During the administration period, urinary protein was measured once a week to examine positive ratio in each week of age by defining an individual showing a urinary protein level of 100 mg/dl or more as positive. Also, blood urea nitrogen was measured on the next day of the final administration to carry out serologic biochemical evaluation of the action of the compound of the present invention against nephritis, together with histopathological evaluation.

### III) Results and discussion

As shown in Table 1, it was revealed that increment of proteinuria was suppressed in the inventive compound-administered groups, whereas increment of proteinuria was observed in the untreated group starting on 15 to 16 weeks of age.

The compound of the present invention also suppressed blood urea significantly, thus clearly showing its suppression action against nephritis in MRL/1 mice.

**Table 1 protein**

| Changes in positive urinary protein | | | | |
|---|---|---|---|---|
| | Dose (mg/kg) | Weeks of Age | | |
| | | 16 | 17 | 18 |
| | | | | |

| a) Sulfated glycolipid of biological origin | | | | |
|---|---|---|---|---|
| (1) sulfatide | 2 | 2/15 | 5/15 | 9/15 |
| (2) seminolipid | 2 | 3/15 | 4/15 | 8/15 |
| (3) SM3 | 2 | 2/15 | 4/15 | 8/15 |
| (4) SB2 | 2 | 4/15 | 5/15 | 10/15 |
| (5) SB1a | 2 | 5/15 | 7/15 | 10/15 |

| b) Fucosylated glycolipid of biological origin | | | | |
|---|---|---|---|---|
| (1) sialyl Lewis X | 1 | 3/15 | 4/15 | 8/15 |
| (2) sialyl Lewis A | 1 | 3/15 | 5/14 | 9/14 |

| c) Synthetic sulfated saccharide | | | | |
|---|---|---|---|---|
| (1) chondroitin sulfate | 5 | 4/15 | 7/15 | 10/15 |
| (2) heparitin sulfate | 5 | 4/15 | 8/15 | 11/14 |
| (3) keratan sulfate | 5 | 5/15 | 8/15 | 10/15 |
| (4) dextran sulfate | 5 | 5/15 | 7/15 | 10/15 |
| (5) galactose sulfate | 5 | 3/15 | 5/15 | 8/15 |
| d) Nontreated group | | 7/15 | 9/15 | 13/15 |
| (For example, 7/15 means 7 of 15 animals were positive, and both numerators and denominators are survived animals) | | | | |

### PHARMACOLOGICAL DRUG EFFECT TEST EXAMPLE 2 (HEPATIC TOXICITY REDUCING EFFECT)

Using rats of 10 months of age (8 to 9 animals per 1 group), 0.5 g/kg of carbon tetrachloride was orally administered as a hepatic toxicity substance to increase the serum GOT level to 500 KARMEN units or more after 24 hours of the administration, simultaneously carrying out oral administration of (1) indomethacin (5 mg/kg) or (2) tetracycline (10 mg/kg) as a pharmaceutical drug having hepatic toxicity, 3 times on every other day.

In the inventive substance-treated group, the sulfatide composition described in Composition Production Example (2 mg/kg as sulfatide) was orally administered simultaneously with the pharmaceutical drug having hepatic toxicity.

The GOT enzyme released in blood caused by hepatocyte destruction was measured 12 hours after completion of the drug administration, and the liver was extracted to carry out histological tissue examination.

As shown in Table 2, while increase in the GOT value of 800 KARMEN units or more was observed in the test group, the inventive substance-administered group showed improved effect or no turning for the worse even when the hepatotoxic drug was administered.

**Table 2**

| Changes in GOT | | |
|---|---|---|
| Group | 24 hrs after Administration of Carbon Tetrachloride | 12 hrs after Completion of Drug Administration |
| a) | 536 ± 54.7 | 1054 ± 31.2 |
| b) | 536 ± 54.7 | 998 ± 24.1 |
| c) | 536 ± 54.7 | 469 ± 61.5 |
| d) | 536 ± 54.7 | 439 ± 30.5 |
| e) | 536 ± 54.7 | 128 ± 48.7 |
| Description of the group: a) dosed with carbon tetrachloride + (1) indomethacin b) dosed with carbon tetrachloride + (2) tetracycline c) dosed with carbon tetrachloride + (1) indomethacin + sulfatide d) dosed with carbon tetrachloride + (2) tetracycline + sulfatide e) dosed with carbon tetrachloride + sulfatide | | |

### PHARMACOLOGICAL DRUG EFFECT TEST EXAMPLE 3 (EFFECT AS AN INTERFERON AS AN INTERFERON ENHANCER - 1)

Synergetic effect of the simultaneous use of interferon-α (IFN-α) and the substance of the present invention, sulfatide, on mice infected with mouse hepatitis virus (MHV)

### (1) Method

A total of 10 ICR mice were used in one group, and intraperitoneal administration of mouse IFN-α and oral administration of propagermanium were carried out once a day for 3 days. Just before the administration on the second day, MHV Friend strain was inoculated into the abdominal cavity in a 2 LD₅₀ equivalent dose, and conditions of the animals were observed until 14 days thereafter to calculate survival ratio.

### (2) Results and discussion

Significant prolongation effect of average survival days was observed by single administration of 4 × 10⁴ IU/kg IFN-α, and the effect of IFN-α was further increased significantly in a dose dependent fashion by the simultaneous use of sulfatide.

### PHARMACOLOGICAL DRUG EFFECT TEST EXAMPLE 4 (EFFECT AS AN INTERFERON AS AN INTERFERON ENHANCER - 1)

Synergetic effect of the simultaneous use of IFN-α and sulfatide on mice infected with herpes simplex type 1 virus (HSV-1)

### (1) Method

A total of 10 BALB/c mice were used in one group and infected with a 10 LD₅₀ equivalent dose of HSV-1 MIYAMA strain. Intraperitoneal administration of IFN-α was carried out on the day before the inoculation of HSV-1 and just after the inoculation, and oral administration of sulfatide was carried out continuously for 5 days starting on the day before the HSV-1 inoculation until the fourth day after the inoculation. Conditions of the animals were observed until 20 days after the HSV-1 infection to calculate survival rate.

### (2) Results and discussion

Though IFN-α showed no significant effect by its single administration in a dose of 4 × 10⁴ IU/kg, sulfatide was significantly effective in dose-dependently prolonging average survival days when used together with 4 × 10⁴ IU/kg of IFN-α.

The present invention provides an agent for use in the suppression of cell degeneration and reduction of organ toxicity, which is useful as the preventive and therapeutic agent of cell degeneration and organ disorders caused by poisoning accidents and viral infection and can reduce side effects of drugs for use in the treatment of complications such as infectious diseases and cancers, when such complications occur in patients who are apt to receive side effects due to reduced functions in the liver, kidney and the other organs, cerebral tissues and cells or who have a narrow range of the application dose of medicines like the case of pregnant women, babies, the aged and the like, so that such drugs can be administered safely to these patients.

While the invention has been described in detail and with reference to specific embodiments thereof, it will be apparent to one skilled in the art that various changes and modifications can be made therein without departing from the spirit and scope thereof.

## Claims

1. A pharmaceutical composition for suppressing cell degeneration and reducing organ toxicity, which comprises as a main ingredient an inhibitor for a neutrophil surface-adhering molecule selectin.

2. The pharmaceutical composition for suppressing cell degeneration and reducing organ toxicity of Claim 1, wherein said selectin inhibitor comprises a sulfated glycolipid or fucosylated glycolipid of biological origin, a synthetic sulfated saccharide, or a mixture composition thereof.

3. The pharmaceutical composition for suppressing cell degeneration and reducing organ toxicity of Claim 1, wherein said selectin inhibitor comprises a sulfated glycolipid or fucosylated glycolipid of biological origin, a synthetic sulfated saccharide, or a mixture composition thereof.

4. The pharmaceutical composition for suppressing cell degeneration and reducing organ toxicity of Claim 3, wherein said sulfated glycolipid of biological origin is selected from the group consisting of (1) sulfatide, (2) seminolipid, (3) SM3 (II³ SO₃ Lac Cer), (4) SB2 (III³ II³ SO₃ Ge3Cer) and (5) SB1a (IV³ SO₃ II³ SO₃ Ge4Cer).

5. The pharmaceutical composition for suppressing cell degeneration and reducing organ toxicity of Claim 3, wherein said fucosylated glycolipid is selected from the group consisting of(1) sialyl Lewis X (SLe^{x}) and (2) sialyl Lewis A (SLe^{a}).

6. The pharmaceutical composition for suppressing cell degeneration and reducing organ toxicity of Claim 3, wherein said synthetic sulfated saccharide is selected from the group consisting of (1) chondroitin sulfate, (2) heparitin sulfate, (3) keratan sulfate, (4) dextran sulfate and (5) galactose sulfate.

7. Use of an inhibitor for a neutrophil surface-adhering molecule selectin for the manufacture of a medicament for suppressing cell degeneration and reducing organ toxicity.

8. Use of an inhibitor for a neutrophil surface-adhering molecule selectin for the manufacture of a medicament for prolonging the term in which a medicine having high toxicity can be administered.

9. Use of an inhibitor for a neutrophil surface-adhering molecule selectin for the manufacture of a medicament for suppressing of renal disorders caused by a substance having renal toxicity.

10. Use of an inhibitor for a neutrophil surface-adhering molecule selectin for the manufacture of a medicament for suppressing hepatic disorders caused by a substance having hepatic toxicity.

11. Use of an inhibitor for a neutrophil surface-adhering molecule selectin for the manufacture of a medicament for suppressing nervous disorders caused by a substance having cranial nerve system toxicity.

12. Use of an inhibitor for a neutrophil surface-adhering molecule selectin for the manufacture of a medicament for reducing organ disorders induced by a microorganism as the causative agent.

13. Use of an inhibitor for a neutrophil surface-adhering molecule selectin for the manufacture of a medicament for suppressing renal disorders caused by administration of indomethacin-based analgesics or kanamycin-based antibiotics.

14. Use of an inhibitor for a neutrophil surface-adhering molecule selectin for the manufacture of a medicament for suppressing hepatic and renal disorders caused by administration of 5-fluorouracil or cisplatin-based anticancer drugs.

15. Use of an inhibitor for a neutrophil surface-adhering molecule selectin for the manufacture of a medicament for suppressing nervous disorders caused by mercury or other heavy metal substances.

16. Use of an inhibitor for a neutrophil surface-adhering molecule selectin for the manufacture of a medicament for suppressing hepatic disorders caused by carbon tetrachloride or chronic alcoholism.

17. Use of an inhibitor for a neutrophil surface-adhering molecule selectin for the manufacture of a medicament for reducing hepatic disorders caused by infection with A type, B type, C type or non-A non-B non-C type hepatitis virus.

18. Use of an inhibitor for a neutrophil surface-adhering molecule selectin for the manufacture of a medicament for suppressing immunodeficiencies and cranial nerve disorders caused by infection with a retroviruse.
